# EUROPEAN PATENT APPLICATION

(11) **EP 1 374 909 A2**
(43) Date of publication of application: **02.01.2004**
(21) Application number: 03012035.6
(22) Date of filing: 20.07.1998
(51) Int. Cl.: A61K 48/00

(54) **Evaluation of, delivery of, and use of agents to treat heart disorders**

(30) Priority: 22.07.1997 US 53356 P
(62) Divisional of application: 98934656.4
(71) Applicant: THE GENERAL HOSPITAL CORPORATION, Boston, MA 02110 (US)
(72) Inventor: Rosenzweig, Anthony, Newton, Massachusetts 02168 (US); Guerrero, Luis, Norton, Massachusetts 02766 (US); Hajjar, Roger J., Cambridge, Massachusetts 02141 (US)
(74) Representative: Ruffles, Graham Keith

(57) **Abstract**

The invention provides methods of evaluating a treatment for a heart disorder. The invention also provides methods of delivering a compound to the heart of a subject. A heart cell, into which has been introduced by somatic gene transfer, a nucleic acid which results in the expression of phospholamban, a heart tissue, into which has been introduced by somatic gene transfer, a nucleic acid which results in the expression of phospholamban and a heart, into which has been introduced by somatic gene transfer, a nucleic acid which results in the expression of phospholamban are also provided by the invention. Finally, the invention provides a method for treating a subject at risk for a heart disorder.

## Description

### Field of the Invention

This invention relates to methods for evaluating treatments suitable for heart disorders and to methods for delivering compounds to the heart of a subject.

### Background of the Invention

The sarcoplasmic reticulum (SR) is an internal membrane system, which plays a critical role in the regulation of cytosolic Ca²⁺ concentrations and thus, excitation-contraction coupling in muscle. Contraction is mediated through the release of Ca²⁺ from the SR, while relaxation involves the active re-uptake of Ca²⁺ into the SR lumen by a Ca²⁺ -ATPase. In cardiac muscle, the SR Ca²⁺ -ATPase activity (SERCA2a) is under reversible regulation by phospholamban.

Phospholamban is a small phosphoprotein, about 6,080 daltons in size, which is an integral element of the cardiac SR membrane. Phospholamban is phosphorylated in vivo in response to β-adrenergic agonist stimulation. In the dephosphorylated state, phospholamban inhibits SR Ca²⁺ -ATPase activity by decreasing the affinity of the enzyme for Ca²⁺.

Heart failure is characterized by a number of abnormalities at the cellular level in the various steps of excitation-contraction coupling of the cardiac cells. One of the key abnormalities in both human and experimental heart failure is a defect in SR function which is associated with abnormal intracellular Ca²⁺ handling. Deficient SR Ca²⁺ uptake during relaxation has been identified in failing hearts from both humans and animal models and has been associated with a decrease in the activity of SR Ca²⁺-ATPase activity and altered Ca²⁺ kinetics.

### Summary of the Invention

In general, the invention features, a method of evaluating a treatment for a heart disorder. The method includes:
providing a heart cell, into which has been introduced by somatic gene transfer, a nucleic acid which results in the expression of phospholamban; administering the treatment to the heart cell; and evaluating the effect of the treatment on the heart cell, thereby evaluating the treatment for a heart disorder.

In preferred embodiments, the method includes evaluating the effect of the treatment on a parameter related to heart function. The parameter, by way of example, can include an assessment of contractility, Ca²⁺ metabolism, e.g., intracellular Ca²⁺ concentration, SR Ca²⁺ ATPase activity, force generation, a force frequency relationship, cardiocyte survival or apoptosis or ion channel activity, e.g., sodium calcium exchange, sodium channel activity, calcium channel activity, or sodium potassium ATPase pump activity.

In preferred embodiments, the treatment is administered *in vivo,* e.g., to an experimental animal. The experimental animal can be an animal in which a gene related to cardiac structure or function is misexpressed. Misexpression can be achieved by methods known in the art, for example, by transgenesis, including the creation of knockout animals, or by classic breeding experiments or manipulation. The misexpressed gene can be a gene encoding a sarcomeric protein, a gene encoding a protein which conditions cardiocyte survival or apoptosis, or a gene encoding a calcium regulatory protein. Sarcomeric proteins include myosin heavy chain, troponin I, troponin C, troponin T, tropomyosin, actin, myosin light chain kinase, myosin light chain 1, myosin light chain 2 or myosin light chain 3. Proteins which modify cardiocyte survival or apoptosis include IGF-1 receptor, PI₃ kinase, AKT kinase or members of the caspase family of proteins. Calcium regulatory proteins include phospholamban, SR Ca2⁺ ATPase, sodium-calcium exchanger, calcium channel (L and T), calsequestrin or calreticulin. The experimental animal can be an animal model for a disorder, e.g., a heart disorder.

In preferred embodiments, the treatment is administered *in vitro.* In preferred embodiments the cell is derived from an experimental animal or a human. In preferred embodiments the cell can be cultured and/or immortalized.

In preferred embodiments, the nucleic acid encodes a phospholamban protein. The phospholamban can be from the same species that the heart cell is from or it can be from a different species. For example, a mouse phospholamban can be expressed in a mouse cell or a human phospholamban can be expressed in a cell from an experimental animal.

In preferred embodiments, the nucleic acid is introduced into the heart cell by way of a vector suitable for somatic gene transfer, e.g., a viral vector, e.g., an adenoviral vector.

In another aspect, the invention features, a method of evaluating a treatment for a heart disorder. The method includes: providing a heart, into some or all the cells of which has been introduced, by somatic gene transfer, a nucleic acid which results in the expression of phospholamban; administering the treatment to the heart; and evaluating the effect of the treatment on the heart, thereby evaluating the treatment for a heart disorder.

In preferred embodiments, the method includes evaluating the effect of the treatment on a parameter related to heart function. The parameter, by way of example, can include an assessment of contractility, Ca²⁺ metabolism, e.g., intracellular Ca²⁺ concentration, SR Ca²⁺ ATPase activity, force generation, a force frequency relationship, cardiocyte survival or apoptosis or ion channel activity, e.g., sodium calcium exchange, sodium channel activity, calcium channel activity, or sodium potassium ATPase pump activity.

In preferred embodiments, the treatment is administered *in vivo,* e.g., to an experimental animal. The experimental animal can be an animal in which a gene related to cardiac structure or function is misexpressed. Misexpression can be achieved by methods known in the art, for example, by transgenesis, including the creation of knockout animals, or by classic breeding experiments or manipulation. The misexpressed gene can be a gene encoding a sarcomeric protein, a gene encoding a protein which conditions cardiocyte survival or apoptosis, or a gene encoding a calcium regulatory protein. Sarcomeric proteins include myosin heavy chain, troponin I, troponin C, troponin T, tropomyosin, actin, myosin light chain kinase, myosin light chain 1, myosin light chain 2 or myosin light chain 3. Proteins which modify cardiocyte survival or apoptosis include IGF-1 receptor, PI₃ kinase, AKT kinase or members of the caspase family of proteins. Calcium regulatory proteins include phospholamban, SR Ca2⁺ ATPase, sodium-calcium exchanger, calcium channel (L and T), calsequestrin or calreticulin. The experimental animal can be an animal model for a disorder, e.g., a heart disorder.

In preferred embodiments, the treatment is administered *in vitro.* In preferred embodiments the heart is derived from an experimental animal or a human.

In preferred embodiments, the nucleic acid encodes a phospholamban protein. The phospholamban can be from the same species that the heart is from or it can be from a different species. For example, a mouse phospholamban can be expressed in a mouse heart or a human phospholamban can be expressed in the heart of an experimental animal. The phospholamban can be delivered to the heart using methods described herein.

In preferred embodiments, the nucleic acid is introduced into the heart by way of a vector suitable for somatic gene transfer, e.g., a viral vector, e.g., an adenoviral vector.

In another aspect, the invention features, a method of evaluating a treatment for a heart disorder. The method includes: providing heart tissue into some or all of the cells of which has been introduced, by somatic gene transfer, a nucleic acid which results in the expression of phospholamban; administering the treatment to the heart tissue; and evaluating the effect of the treatment on the heart tissue, thereby evaluating the treatment for a heart disorder.

In preferred embodiments, the method includes evaluating the effect of the treatment on a parameter related to heart function. The parameter, by way of example, can include an assessment of contractility, Ca²⁺ metabolism, e.g., intracellular Ca²⁺ concentration, SR Ca²⁺ ATPase activity, force generation, a force frequency relationship, cardiocyte survival or apoptosis or ion channel activity, e.g., sodium calcium exchange, sodium channel activity, calcium channel activity, or sodium potassium ATPase pump activity.

In preferred embodiments, the treatment is administered *in vivo,* e.g., to an experimental animal. The experimental animal can be an animal in which a gene related to cardiac structure or function is misexpressed. Misexpression can be achieved by methods known in the art, for example, by transgenesis, including the creation of knockout animals, or by classic breeding experiments or manipulation. The misexpressed gene can be a gene encoding a sarcomeric protein, a gene encoding a protein which conditions cardiocyte survival or apoptosis, or a gene encoding a calcium regulatory protein. Sarcomeric proteins include myosin heavy chain, troponin I, troponin C, troponin T, tropomyosin, actin, myosin light chain kinase, myosin light chain 1, myosin light chain 2 or myosin light chain 3. Proteins which modify cardiocyte survival or apoptosis include IGF-1 receptor, PI₃ kinase, AKT kinase or members of the caspase family of proteins. Calcium regulatory proteins include phospholamban, SR Ca2⁺ ATPase, sodium-calcium exchanger, calcium channel (L and T), calsequestrin or calreticulin. The experimental animal can be an animal model for a disorder, e.g., a heart disorder.

In preferred embodiments, the treatment is administered *in vitro.* In preferred embodiments the heart tissue is derived from an experimental animal or a human.

In preferred embodiments, the nucleic acid encodes a phospholamban protein. The phospholamban can be from the same species that the heart tissue is from or it can be from a different species. For example, a mouse phospholamban can be expressed in a mouse heart tissue or a human phospholamban can be expressed in heart tissue from an experimental animal.

In preferred embodiments, the nucleic acid is introduced into the heart tissue by way of a vector suitable for somatic gene transfer, e.g., a viral vector, e.g., an adenoviral vector.

In another aspect, the invention features, a method of evaluating a treatment for a heart disorder. The method includes: providing a first and a second heart cell, into each of which has been introduced, by somatic gene transfer, a nucleic acid which results in the expression of phospholamban; administering the treatment to a first heart cell, preferably in vitro; evaluating the effect of the treatment on the first heart cell; administering the treatment to a second heart cell, preferably in vivo; and evaluating the effect of the treatment on the second heart cell, thereby evaluating the treatment for a heart disorder.

In preferred embodiments, the method includes evaluating the effect of the treatment on a parameter related to heart function. The parameter, by way of example, can include an assessment of contractility, Ca²⁺ metabolism, e.g., intracellular Ca²⁺ concentration, SR Ca²⁺ ATPase activity, force generation, a force frequency relationship, cardiocyte survival or apoptosis or ion channel activity, e.g., sodium calcium exchange, sodium channel activity, calcium channel activity, or sodium potassium ATPase pump activity.

In preferred embodiments, the treatment is administered *in vivo,* e.g., to an experimental animal. The experimental animal can be an animal in which a gene related to cardiac structure or function is misexpressed. Misexpression can be achieved by methods known in the art, for example, by transgenesis, including the creation of knockout animals, or by classic breeding experiments or manipulation. The misexpressed gene can be a gene encoding a sarcomeric protein, a gene encoding a protein which conditions cardiocyte survival or apoptosis, or a gene encoding a calcium regulatory protein. Sarcomeric proteins include myosin heavy chain, troponin I, troponin C, troponin T, tropomyosin, actin, myosin light chain kinase, myosin light chain 1, myosin light chain 2 or myosin light chain 3. Proteins which modify cardiocyte survival or apoptosis include IGF-1 receptor, PI₃ kinase, AKT kinase or members of the caspase family of proteins. Calcium regulatory proteins include phospholamban, SR Ca2⁺ ATPase, sodium-calcium exchanger, calcium channel (L and T), calsequestrin or calreticulin. The experimental animal can be an animal model for a disorder, e.g., a heart disorder.

In preferred embodiments, the nucleic acid encodes a phospholamban protein. The phospholamban can be from the same species that the heart cell is from or it can be from a different species. For example, a mouse phospholamban can be expressed in a mouse cell or a human phospholamban can be expressed in a cell from an experimental animal.

In preferred embodiments, the first and second cell can be from the same or different animals, can be from the same or different species, e.g., the first cell can be from a mouse and the second cell can be from a human or both cells can be human. The first and second cell can have the same or different genotypes. In further preferred embodiments the evaluation of the treatment in the first cell can be the same or different from the evaluation of the treatment in the second cell, e.g., the intracellular Ca²⁺ concentration can be measured in the first cell and the SR Ca²⁺ -ATPase activity can be measured in the second cell or the intracellular Ca²⁺ concentration can be measured in both cells.

In another aspect, the invention features, a method of evaluating a treatment for a heart disorder. The method includes: providing a first administration of a treatment to a heart cell, into which has been introduced by somatic gene transfer, a nucleic acid which results in the expression of phospholamban; evaluating the effect of the first administration on the heart cell; providing a second administration of a treatment to a heart cell, into which has been introduced by somatic gene transfer, a nucleic acid which results in the expression of phospholamban; and evaluating the effect of the second administration on the heart cell, thereby evaluating a treatment for a heart disorder.

In preferred embodiments, the method includes evaluating the effect of the treatment on a parameter related to heart function. The parameter, by way of example, can include an assessment of contractility, Ca²⁺ metabolism, e.g., intracellular Ca²⁺ concentration, SR Ca²⁺ ATPase activity, force generation, a force frequency relationship, cardiocyte survival or apoptosis or ion channel activity, e.g., sodium calcium exchange, sodium channel activity, calcium channel activity, or sodium potassium ATPase pump activity.

In preferred embodiments, the treatment is administered *in vivo,* e.g., to an experimental animal. The experimental animal can be an animal in which a gene related to cardiac structure or function is misexpressed. Misexpression can be achieved by methods known in the art, for example, by transgenesis, including the creation of knockout animals, or by classic breeding experiments or manipulation. The misexpressed gene can be a gene encoding a sarcomeric protein, a gene encoding a protein which conditions cardiocyte survival or apoptosis, or a gene encoding a calcium regulatory protein. Sarcomeric proteins include myosin heavy chain, troponin I, troponin C, troponin T, tropomyosin, actin, myosin light chain kinase, myosin light chain 1, myosin light chain 2 or myosin light chain 3. Proteins which modify cardiocyte survival or apoptosis include IGF-1 receptor, PI₃ kinase, AKT kinase or members of the caspase family of proteins. Calcium regulatory proteins include phospholamban, SR Ca2⁺ ATPase, sodium-calcium exchanger, calcium channel (L and T), calsequestrin or calreticulin. The experimental animal can be an animal model for a disorder, e.g., a heart disorder.

In preferred embodiments, the nucleic acid encodes a phospholamban protein. The phospholamban can be from the same species that the heart cell is from or it can be from a different species. For example, a mouse phospholamban can be expressed in a mouse cell or a human phospholamban can be expressed in a cell from an experimental animal.

In preferred embodiments the first and second administration can be administered to the same or to different cells. The first and second administration can be administered under the same or different conditions, e.g., the first administration can consist of a relatively low level treatment, e.g., a lower concentration of a substance, and the second administration can consist of a relatively high level treatment, e.g., a higher concentration of a substance or both administrations can consist of the same level treatment.

In another aspect, the invention features, a method of evaluating a treatment for a heart disorder. The method includes: providing a heart cell, into which has been introduced by somatic gene transfer, a nucleic acid which results in the expression of phospholamban; administering the treatment to the heart cell; evaluating the effect of the treatment on the heart cell; providing a heart, into which has been introduced by somatic gene transfer, a nucleic acid which results in the expression of phospholamban; administering the treatment to the heart ; and evaluating the effect of the treatment on the heart, thereby evaluating the treatment for a heart disorder.

In preferred embodiments, the method includes evaluating the effect of the treatment on a parameter related to heart function. The parameter, by way of example, can include an assessment of contractility, Ca²⁺ metabolism, e.g., intracellular Ca²⁺ concentration, SR Ca²⁺ ATPase activity, force generation, a force frequency relationship, cardiocyte survival or apoptosis or ion channel activity, e.g., sodium calcium exchange, sodium channel activity, calcium channel activity, or sodium potassium ATPase pump activity.

In preferred embodiments, the treatment is administered *in vivo*, e.g., to an experimental animal. The experimental animal can be an animal in which a gene related to cardiac structure or function is misexpressed. Misexpression can be achieved by methods known in the art, for example, by transgenesis, including the creation of knockout animals, or by classic breeding experiments or manipulation. The misexpressed gene can be a gene encoding a sarcomeric protein, a gene encoding a protein which conditions cardiocyte survival or apoptosis, or a gene encoding a calcium regulatory protein. Sarcomeric proteins include myosin heavy chain, troponin I, troponin C, troponin T, tropomyosin, actin, myosin light chain kinase, myosin light chain 1, myosin light chain 2 or myosin light chain 3. Proteins which modify cardiocyte survival or apoptosis include IGF-1 receptor, PI₃ kinase, AKT kinase or members of the caspase family of proteins. Calcium regulatory proteins include phospholamban, SR Ca2⁺ ATPase, sodium-calcium exchanger, calcium channel (L and T), calsequestrin or calreticulin. The experimental animal can be an animal model for a disorder, e.g., a heart disorder.

In preferred embodiments, the nucleic acid encodes a phospholamban protein. The phospholamban can be from the same species that the heart cell and/or the heart is from or it can be from a different species. For example, a mouse phospholamban can be expressed in a mouse heart cell and/or heart or a human phospholamban can be expressed in a heart cell and/or heart from an experimental animal.

In preferred embodiments the treatment can be administered to the heart cell in vitro and to the heart in vivo or the treatment can be administered to the heart cell and to the heart in vitro.

In another aspect, the invention features, a method of delivering a compound to the heart of a subject. The method includes: restricting the aortic flow of blood out of the heart, such that blood flow is re-directed to the coronary arteries; introducing the compound into the lumen of the circulatory system such that it flows into the coronary arteries; allowing the heart to pump while the aortic outflow of blood is restricted, thereby allowing the compound to flow into and be delivered to the heart; and reestablishing the flow of blood to the heart .

In preferred embodiments, the compound includes: a nucleic acid which directs the expression of a peptide, e.g., a phospholamban or a SR Ca²⁺ -ATPase and a viral vector suitable for somatic gene delivery, e.g., an adenoviral vector.

In preferred embodiments, the subject is at risk for a heart disorder, e.g., heart failure, ischemia, arrhythmia, myocardial infarction, congestive heart failure, transplant rejection.

In preferred embodiments, the subject can be a human or an experimental animal. The experimental animal can be an animal in which a gene related to cardiac structure or function is misexpressed. Misexpression can be achieved by methods known in the art, for example, by transgenesis, including the creation of knockout animals, or by classic breeding experiments or manipulation. The misexpressed gene can be a gene encoding a sarcomeric protein, a gene encoding a protein which conditions cardiocyte survival or apoptosis, or a gene encoding a calcium regulatory protein. Sarcomeric proteins include myosin heavy chain, troponin I, troponin C, troponin T, tropomyosin, actin, myosin light chain kinase, myosin light chain 1, myosin light chain 2 or myosin light chain 3. Proteins which modify cardiocyte survival or apoptosis include IGF-1 receptor, PI₃ kinase, AKT kinase or members of the caspase family of proteins. Calcium regulatory proteins include phospholamban, SR Ca2⁺ ATPase, sodium-calcium exchanger, calcium channel (L and T), calsequestrin or calreticulin. The experimental animal can be an animal model for a disorder, e.g., a heart disorder.

In preferred embodiments, the method further includes restricting blood flow into the left side of the heart, e.g., by restricting the pulmonary circulation through obstraction of the pulmonary artery, so as to lessen dilution of the compound.

In preferred embodiments the method further includes opening the pericardium and introducing the compound, e.g., using a catheter.

In preferred embodiments, the compound is: introduced into the lumen of the aorta, e.g., the aortic root, introduced into the coronary ostia or introduced into the lumen of the heart.

In preferred embodiments, the nucleic acid, which directs the expression of the peptide, is homogeneously overexpressed in the heart of the subject.

In another aspect, the invention features, a heart cell, into which has been introduced by somatic gene transfer, a nucleic acid which results in the expression of phospholamban. The heart cell can be provided as a purified preparation.

In another aspect, the invention features, a heart tissue, into which has been introduced by somatic gene transfer, a nucleic acid which results in the expression of phospholamban. The heart tissue can be provided as a tissue preparation.

In another aspect, the invention features, a heart, into which has been introduced by somatic gene transfer, a nucleic acid which results in the expression of phospholamban. The heart can be provided in a subject or ex vivo, i.e. removed from a subject.

In another aspect, the invention features, a method for treating a subject at risk for a heart disorder. The method includes introducing into somatic heart tissue of the subject, a nucleic acid which encodes phospholamban.

In preferred embodiments, the nucleic acid is introduced using the methods described herein.

In preferred embodiments, the phospholamban can be from the same species as the subject or it can be from a different species. For example, a human phospholamban can be introduced into a human heart or a human phospholamban can be can be introduced into the heart of an experimental animal.

In preferred embodiments, the nucleic acid is introduced into the heart by way of a vector suitable for somatic gene transfer, e.g., a viral vector, e.g., an adenoviral vector.

In preferred embodiments, the subject can be a human, an experimental animal, e.g., a rat or a mouse, a domestic animal, e.g., a dog, cow, sheep, pig or horse, or a non-human primate, e.g., a monkey. The subject can be suffering from a cardiac disorder, such as heart failure, ischemia, myocardial infarction, congestive heart failure, arrhythmia, transplant rejection and the like.

As used herein, the term "treatment" refers to a procedure (e.g., a surgical method) or the administration of a substance, e.g., a compound which is being evaluated for use in the alleviation or prevention of a heart disorder or symptoms thereof. For example, such treatment can be a surgical procedure, or the administration of a therapeutic agent such as a drug, a peptide, an antibody, an ionophore and the like.

As used herein, the term "heart disorder" refers to a structural or functional abnormality of the heart, that impairs its normal functioning. For example, the heart disorder can be heart failure, ischemia, myocardial infarction, congestive heart failure, arrhythmia, transplant rejection and the like. The term includes disorders characterized by abnormalities of contraction, abnormalities in Ca²⁺ metabolism, and disorders characterized by arrhytmia.

As used herein, the term "heart cell" refers to a cell which can be: (a) part of a heart present in a subject, (b) part of a heart which is maintained *in vitro,* (c) part of a heart tissue, or (d) a cell which is isolated from the heart of a subject. For example, the cell can be a cardiac myocyte.

As used herein, the term "heart" refers to a heart present in a subject or to a heart which is maintained outside a subject.

As used herein, the term "heart tissue" refers to tissue which is derived from the heart of a subject.

As used herein, the term "somatic gene transfer" refers to the transfer of genes into a somatic cell as opposed to transferring genes into the germ line.

As used herein, the term "compound" refers to a compound, which can be delivered effectively to the heart of a subject using the methods of the invention. Such compounds can include, for example, a gene, a drug, an antibiotic, an enzyme, a chemical compound, a mixture of chemical compounds or a biological macromolecule.

As used herein, the term "subject" refers to an experimental animal, e.g., a rat or a mouse, a domestic animal, e.g., a dog, cow, sheep, pig or horse, a non-human primate, e.g., a monkey and in the case of therapeutic methods, humans. However, it is noted that human cells, tissue or hearts can be used in vitro evaluations. A subject can suffer from a heart disorder, such as heart failure, ischemia, myocardial infarction, congestive heart failure, arrhythmia, transplant rejection and the like. The experimental animal can be an animal in which a gene related to cardiac structure or function is misexpressed. Misexpression can be achieved by methods known in the art, for example, by transgenesis, including the creation of knockout animals, or by classic breeding experiments or manipulation. The misexpressed gene can be a gene encoding a sarcomeric protein, a gene encoding a protein which conditions cardiocyte survival or apoptosis, or a gene encoding a calcium regulatory protein. Sarcomeric proteins include myosin heavy chain, troponin I, troponin C, troponin T, tropomyosin, actin, myosin light chain kinase, myosin light chain 1, myosin light chain 2 or myosin light chain 3. Proteins which modify cardiocyte survival or apoptosis include IGF-1 receptor, PI₃ kinase, AKT kinase or members of the caspase family of proteins. Calcium regulatory proteins include phospholamban, SR Ca2⁺ ATPase, sodium-calcium exchanger, calcium channel (L and T), calsequestrin or calreticulin. The experimental animal can be an animal model for a heart disorder, such as a hypertensive mouse or rat.

As used herein, the term "misexpression" refers to a non-wild type pattern of gene expression. It includes: expression at non-wild type levels, i.e., over or under expression; a pattern of expression that differs from wild type in terms of the time or stage at which the gene is expressed, e.g., increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the splicing size, amino acid sequence, post-transitional modification, or biological activity of the expressed polypeptide; a pattern of expression that differs from wild type in terms of the effect of an environmental stimulus or extracellular stimulus on expression of the gene, e.g., a pattern of increased or decreased expression (as compared with wild type) in the presence of an increase or decrease in the strength of the stimulus. Misexpression includes any expression from a transgenic nucleic acid.

As used herein, the term "restricting the aortic flow of blood out of the heart" refers to substantially blocking the flow of blood into the distal aorta and its branches. For example, at least 50 % of the blood flowing out of the heart is restricted, preferably 75 % and more preferably 80, 90, or 100 % of the blood is restricted from flowing out of the heart. The blood flow can be restricted by obstructing the aorta and the pulmonary artery, e.g., with clamps.

As used, herein, the term "introducing" refers to a process by which a compound can be placed into a chamber or the lumen of the heart of a subject. For example, the pericardium can be opened and the compound can be injected into the heart, e.g., using a syringe and a catheter. The compound can be: introduced into the lumen of the aorta, e.g., the aortic root, introduced into the coronary ostia or introduced into the lumen of the heart.

As used herein, the terms "homogeneous fashion" and "homogeneously overexpressing" are satisfied if one or more of the following requirements are met: (a) the compound contacts at least 10 %, preferably 20, 20, 40, 50, 60, 70, 80, 90 or 100 % of the cells of the heart and (b) at least 10 %, preferably 20, 20, 40, 50, 60, 70, 80, 90 or 100 % of the heart cells take up the compound.

As used herein, the term "purified preparation" refers to a preparation in which at least 50, preferably 60, 70, 80 , 90 or 100 % of the cells are heart cells into which phospholamban has been introduced by somatic gene transfer.

The methods of the invention allow rapid and low cost development of cardiac overexpression models. The methods of the invention also provide ways of examining multiple genes interacting in transgenic models, testing gene therapy approaches and evaluating treatments of cardiac disorders.

Other features and advantages of the invention will be apparent from the following detailed description, and from the claims.

### Brief Description of the Drawings

The drawings are first briefly described.

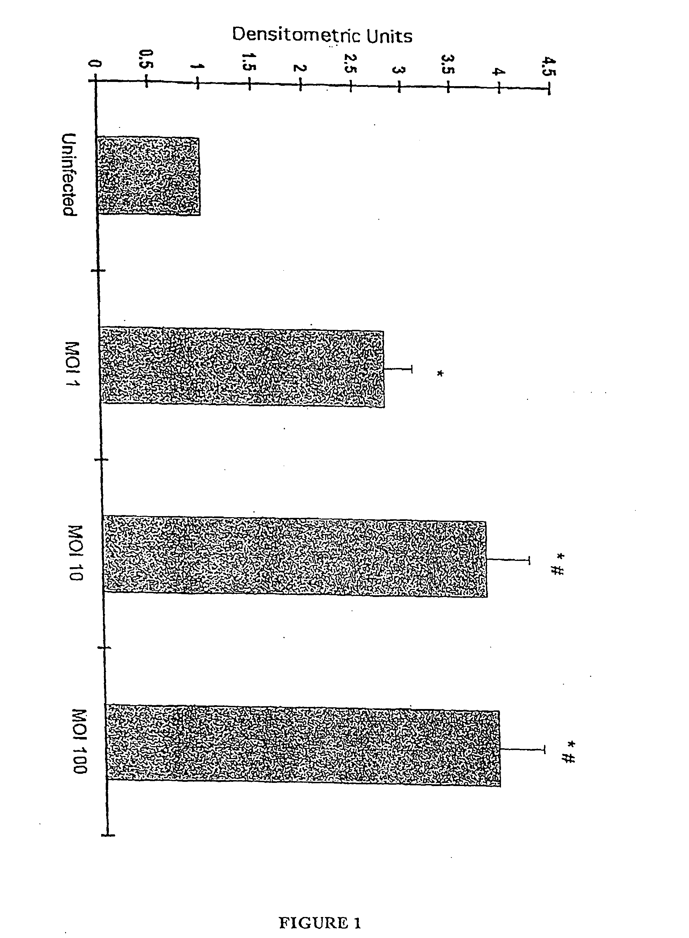

Figure 1 is a graph depicting protein levels of SR Ca²⁺-ATPase in uninfected cardiomyocytes (n=8) and in cardiomyocytes infected for 48 hours with 1, 10, and 100 pfu/cell of Ad.RSV.PL.

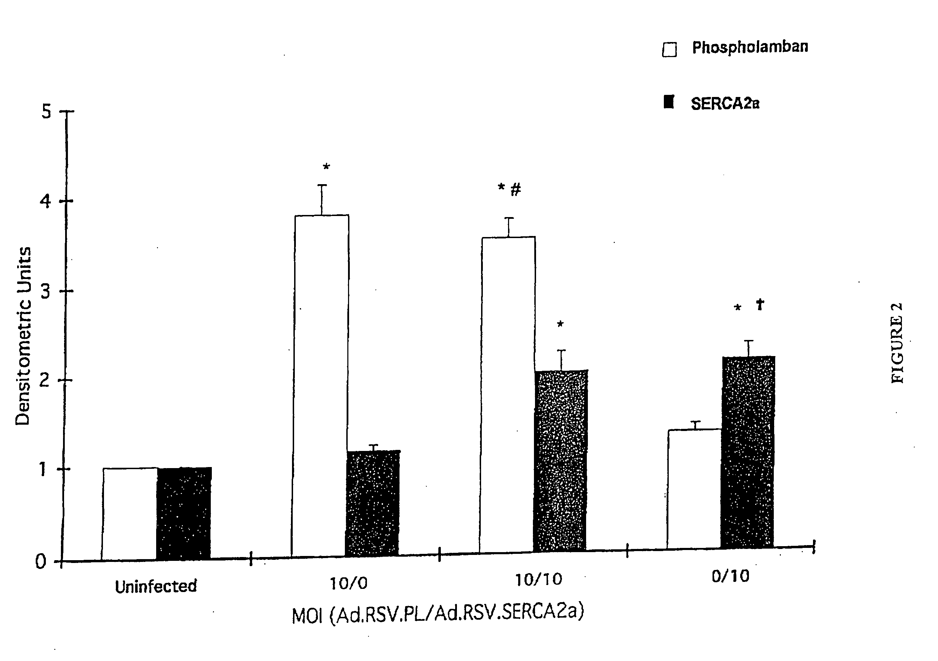

Figure 2 is a graph depicting protein levels of phospholamban and SERCA2a in uninfected cardiomyocytes (n=8) and in cardiomyocytes infected for 48 hours with 10 pfu/cell with either Ad.RSV.PL and/or Ad.RSV.SERCA2a. There were no significant differences between the phospholamban protein levels in the group of myocytes infected with Ad.RSV.PL alone at a multiplicity of infection of 10 pfu/cell and the group of myocytes infected with Ad.RSV.PL at a multiplicity of infection of 10 pfu/cell and Ad.RSV.SERCA2a at a multiplicity of infection of 10 pfu/cell (*P*>2). Similarly, there were no significant differences between the SERCA2a protein levels in the group of myocytes infected with Ad.RSV.SERCA2a alone at a multiplicity of infection of 10 pfu/cell and the group of myocytes infected with Ad.RSV.PL at a multiplicity of infection of 10 pfu/cell and Ad.RSV.SERCA2a at a multiplicity of infection of 10 pfu/cell (*P*>2).

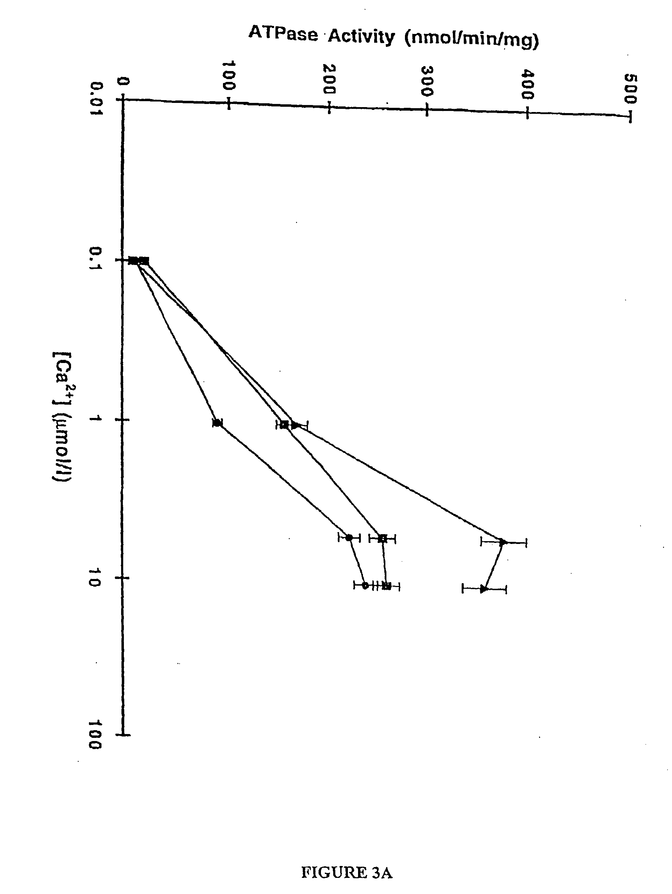

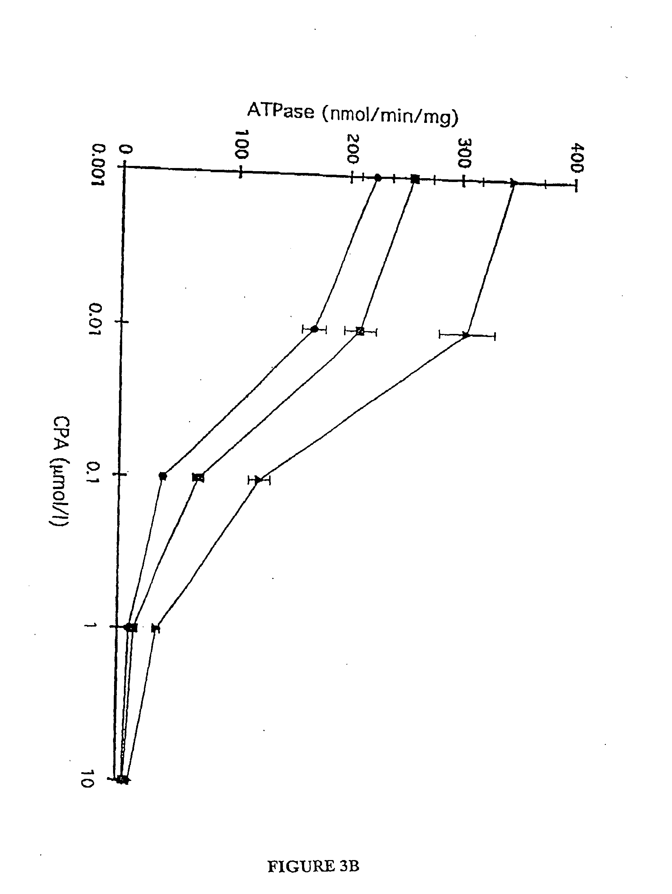

Figure 3A is a graph depicting SERCA2a activity as a function of Ca²⁺ in membrane preparations of uninfected cardiomyocytes (■, n=6), cardiomyocytes infected with 10 pfu/cell of Ad.RSV.PL (•, n=6), and cardiomyocytes infected with 10 pfu/cell of Ad.RSV.PL and 10 pfu/cell of Ad.RSV.SERCA2a (}, n=6). Figure 3B is a graph depicting the effect of increasing concentrations of cyclopiazonic acid (CPA) on SRECA2 activity at a [Ca²⁺] of 10 µmol/L in membrane preparations of uninfected cardiomyocytes (■, n=6), cardiomyocytes infected with 10 pfu/cell of Ad.RSV.PL (•, n=6), and cardiomyocytes infected with 10 pfu/cell of Ad.RSV.PL and 10 pfu/cell of Ad.RSV.SERCA2a (], n=6).

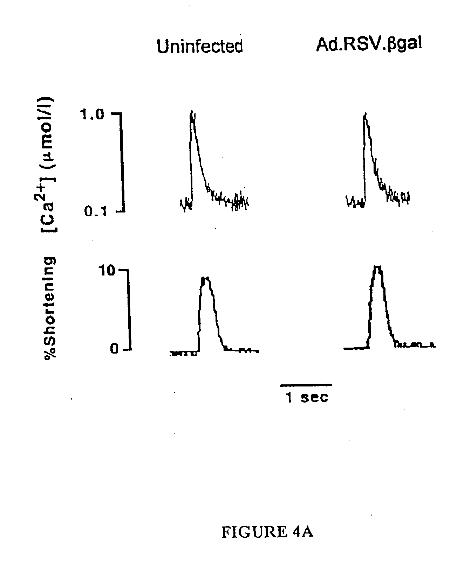

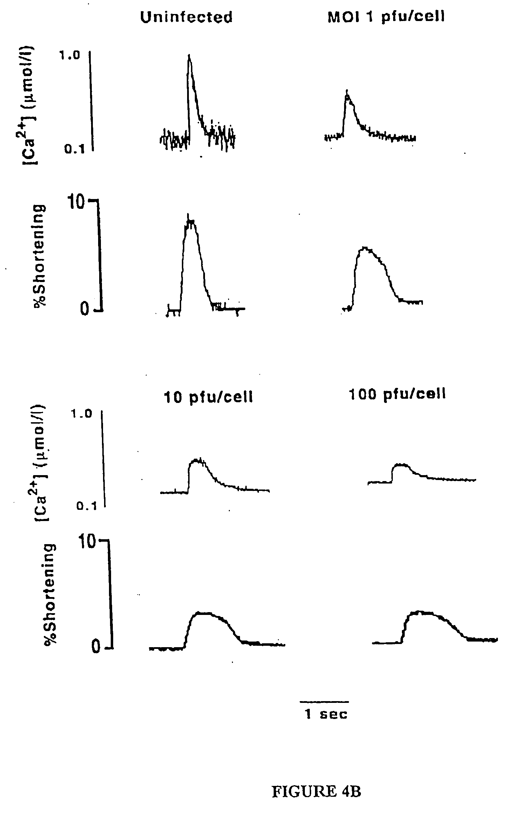

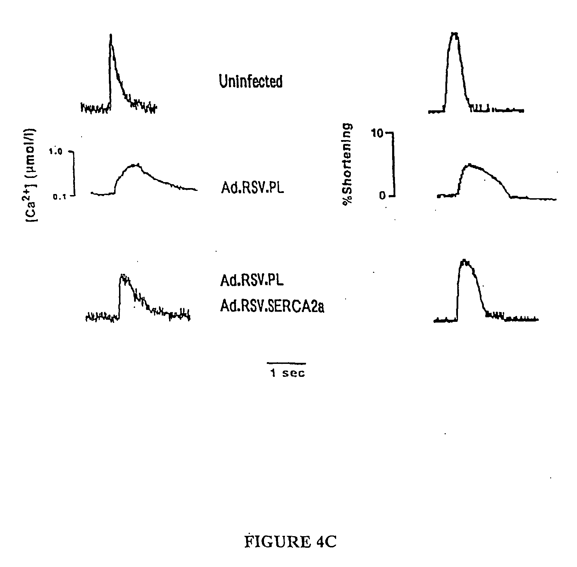

Figure 4A shows intracellular Ca²⁺ transients and shortening in an uninfected cardiomyocyte and in a cardiomyocyte infected for 48 hours with 10 pfu/cell of Ad.RSV.βgal and stimulated at 1 Hz. Figure 4B shows intracellular Ca²⁺ transients and shortening in an uninfected cardiomyocyte and in a cardiomyocyte infected for 48 hours with 1, 10, and 100 pfu/cell of Ad.RSV.PL stimulated at 1 Hz. Figure 4C shows intracellular Ca²⁺ transients and shortening in an uninfected cardiomyocyte, a cardiomyocyte infected with 1- pfu/cell of Ad.RSV.PL, and a cardiomyocyte infected with 10 pfu/cell of Ad.RSV.PL and 10 pfu/cell of Ad.RSV.SERCA2a for 48 hours, stimulated at 1 Hz.

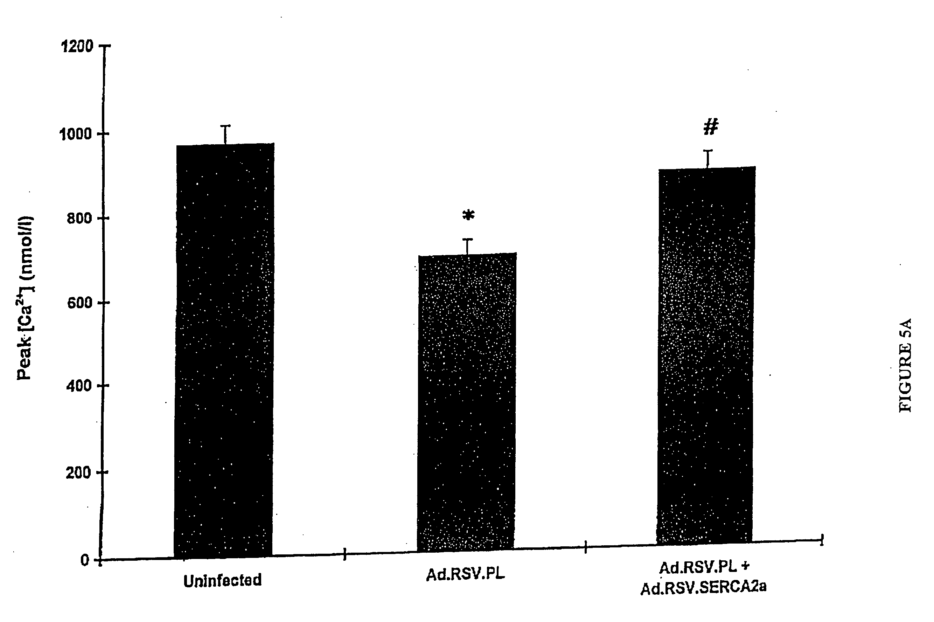

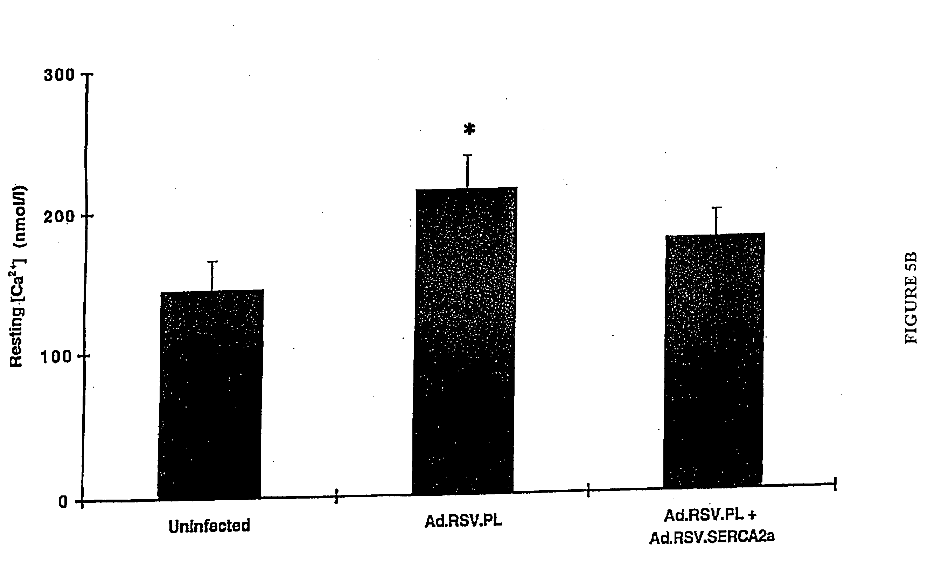

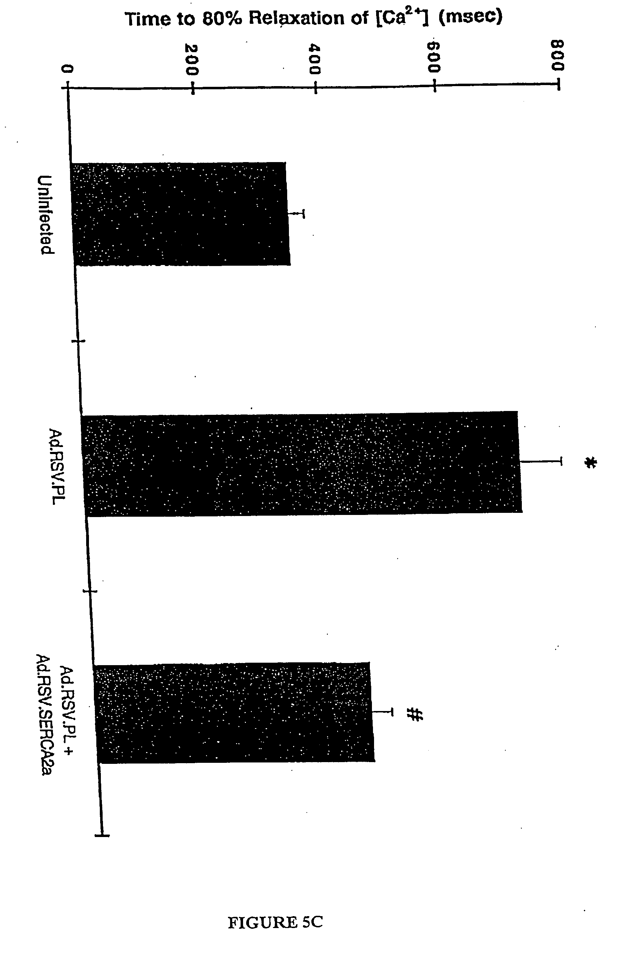

Figure 5A is a graph showing the mean of the peak of the intracellular Ca²⁺ transients in uninfected cardiomyocytes (n=10), cardiomyocytes infected with 10 pfu/cell of Ad.RSV.PL (n=12), and cardiomyocytes infected with 10 pfu/cell of Ad.RSV.PL and 10 pfu/cell of Ad.RSV.SERCA2a (n=10) for 48 hours and stimulated at 1 Hz. Figure 5B is a graph showing the mean of the resting levels of [Ca²⁺] in uninfected cardiomyocytes (n=10), cardiomyocytes infected with 10 pfu/cell of ad.RSV.PL (n=12), and cardiomyocytes infected with 10 pfu/cell of Ad.RSV.PL and 10 pfu/cell of Ad.RSV.SERCA2a (n=10) for 48 hours, stimulated at 1 Hz. Figure 5C is a graph showing the mean of the time to 80% relaxation of the intracellular Ca²⁺ transients in uninfected cardiomyocytes (n=10), cardiomyocytes infected with 10 pfu/cell of Ad.RSV.PL (n=12), and cardiomyocytes infected with 10 pfu/cell of Ad.RSV.PL and 10 pfu/cell of Ad.RSV.SERCA2a (n=10) for 48 hours, stimulated at 1 Hz. *P*<.05 compared with uninfected cells. P<.05 compared with Ad.RSV.PL (multiplicity of infection of 10 pfu/cell).

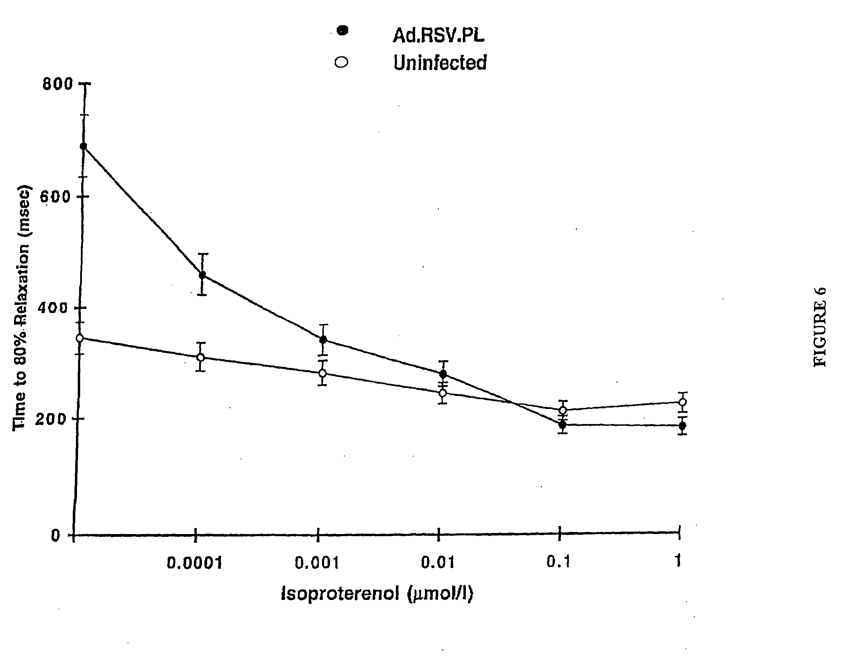

Figure 6 is a graph showing the effect of increasing concentrations of Isoprotenerol on the time course of the intracellular Ca²⁻ transients in uninfected cardiomyocytes (n=5) and cardiomyocytes infected with 10 pfu/cell of Ad.RSV.PL (n=5), stimulated at 1 Hz.

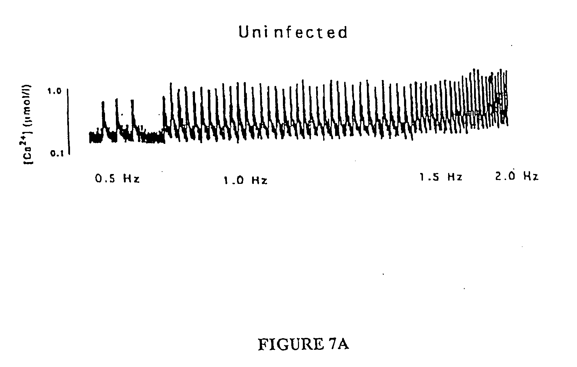

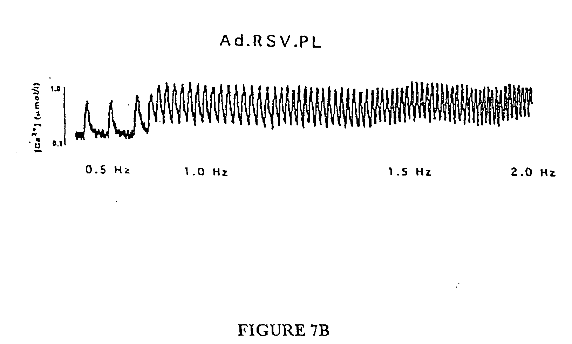

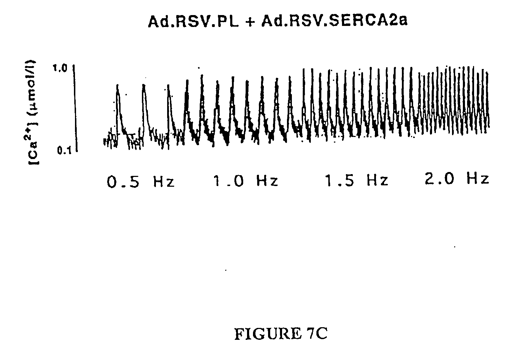

Figure 7A is a graph showing the response of intracellular Ca²⁺ transients to increasing frequency of stimulation in an uninfected cardiomyocyte. Figure 7B is a graph showing the response of intracellular Ca²⁺ transients to increasing frequency of stimulation in a cardiomyocyte infected with 10 pfu/cell of Ad.RSV.PL. Figure 7C is a graph showing the response of intracellular Ca²⁺ transients to increasing frequency of stimulation in a cardiomyocyte infected with 10 pfu/cell of Ad.RSV.PL and 10 pfu/cell of Ad.RSV.SERCA2a for 48 hours.

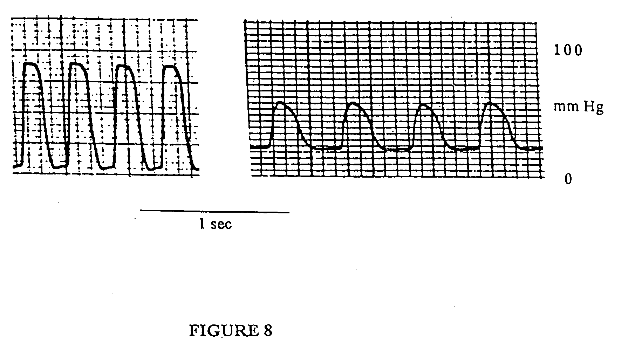

Figure 8 shows intracavitary pressure tracings from rats 48 hours after cardiac gene transfer with either Ad.EGFP (left) or Ad.PL (right). The pressure tracing of the Ad.PL transduced hearts displays a markedly prolonged relaxation and reduced pressure development.

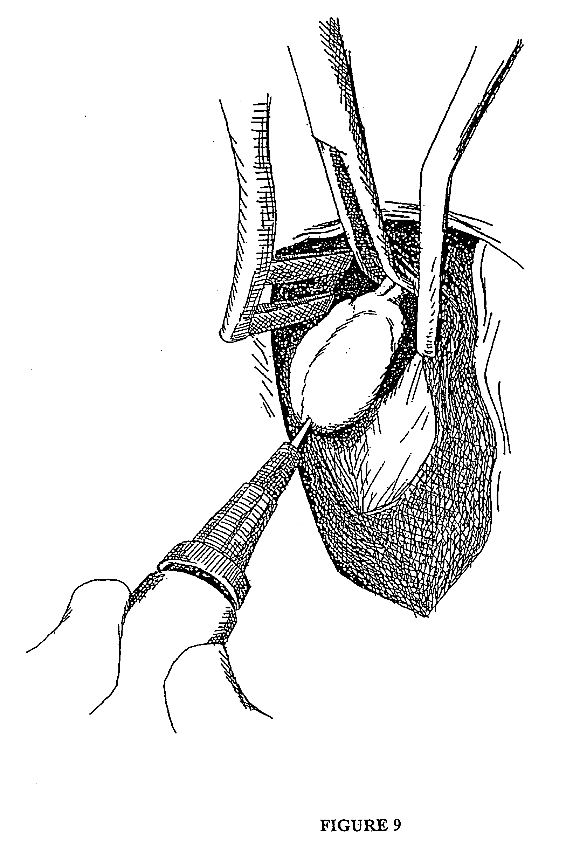

Figure 9 is a drawing showing the somatic gene delivery method.

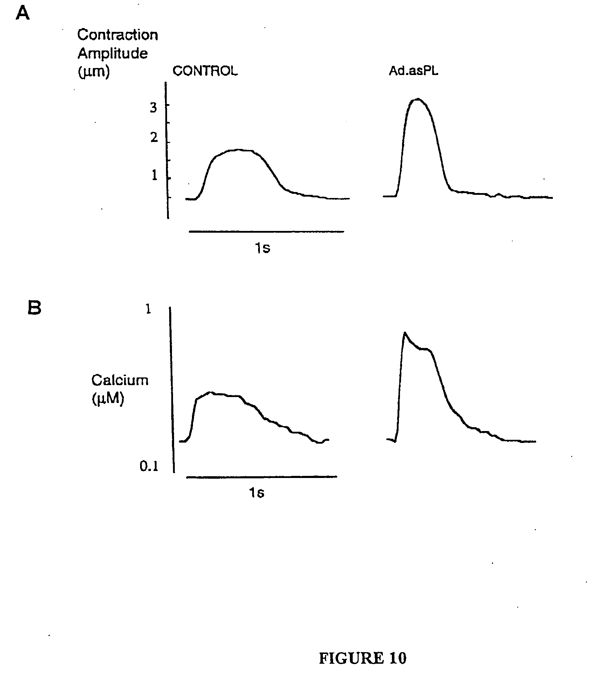

Figure 10A is a graph demonstrating that infection of neonatal cardiac myocytes with the construct Ad.asPL increased the contraction amplitude and significantly shortened the time course of the contraction. Figure 10B is a graph demonstrating that adenovirus-mediated gene transfer of the antisense cDNA for phospholamban results in a modification of intracellular calcium handling.

### Phospholamban and Heart Disorder

The inventors have discovered that somatic gene transfer, e.g., adenoviral gene transfer, is particularly effective in mammalian myocardium both in vivo and in vitro and particularly useful for screening treatments suitable for heart disorders. Adenoviral gene transfer of SERCA2a is both dose dependent and time dependent in rat neonatal cardiomyocytes. An adenovirus encoding phospholamban under the RSV promoter, provided a 4-fold increase in phospholamban, which was also dose dependent. The smaller size of phospholamban compared with SERCA2a (6 kD in its monomer form compared with 110 kD) may explain, at least in part, the more effective protein expression by Ad.RSV.PL than by Ad.RSV.SERCA2a under similar conditions. Nevertheless, using these recombinant adenoviruses, significant overexpression of phospholamban and SERCA2a was achieved, individually and in combination. Coinfection with both Ad.RSV.PL and Ad.RSV.SERCA2a mediated overexpression of both SERCA2a and phospholamban that was the same as the expression from infection with either Ad.RSV.PL or Ad.RSV.SERCA2a alone. The ability to simultaneously manipulate expression of multiple proteins in the context of primary myocytes is an advantage of somatic gene transfer for the study of interacting components of complex systems.

The expression of phospholamban relative to SERCA2a is altered in a number of disease states. In hypothyroidism phospholamban levels are increased, whereas in hyperthyroidism phospholamban levels are decreased. An increased ratio of phospholamban to SERCA2a is an important characteristic of both human and experimental heart failure. Both experimental and human heart failure are characterized by a prolonged Ca²⁺ transient and impaired relaxation. Increasing levels of phospholamban relative to SERCA2a significantly altered intracellular Ca²⁺ handling in the isolated cardiomyocytes by prolonging the relaxation phase of the Ca²⁺ transient, decreasing Ca²⁺ release, and increasing resting Ca²⁺. These results show that altering the relative ratio of phospholamban to SERVA2a can account for the abnormalities in Ca²⁺ handling observed in failing ventricular myocardium. In addition, overexpressing SERCA2a can largely "rescue" the phenotype created by increasing the phospholamban-to-SERCA2a ratio. Restoring the normal phospholamban-to-SERCA2a ratio through somatic gene transfer can correct the abnormalities of Ca²⁺ handling and contraction seen in failing hearts.

### Evaluation of Treatment

A treatment can be evaluated by assessing the effect of the treatment on a parameter related to contractility. For example, SR Ca²⁺ ATPase activity or intracellular Ca²⁺ concentration can be measured, using the methods described above. Furthermore, force generation by hearts or heart tissue can be measured using methods described in Strauss et al., Am. J. Physiol., 262:1437-45, 1992, the contents of which are incorporated herein by reference.

In many drug screening programs which test libraries of therapeutic agents and natural extracts, high throughput assays are desirable in order to maximize the number of therapeutic agents surveyed in a given period of time. Assays which are performed in cell-free systems, such as may be derived with cardiac muscle cell extracts, are preferred as "primary" screens in that they can be generated to permit rapid development and relatively easy detection of the parameter being measured, e.g., the intracellular levels of Ca²⁺, which is mediated by a test therapeutic agent. Moreover, the effects of cellular toxicity and/or bioavailability of the test therapeutic agent can be generally ignored in the *in vitro* system, the assay instead being focused primarily on the effect of the therapeutic agent on the parameter being measured, e.g., the intracellular levels of Ca²⁺. It is often desirable to screen candidate treatments in two stages, wherein the first stage is performed in vitro, and the second stage is performed in vivo.

The efficacy of a test therapeutic agent can be assessed by generating dose response curves from data obtained using various concentrations of the test therapeutic agent. Moreover, a control assay can also be performed to provide a baseline for comparison. In the control assay, the heart cell is incubated in the absence of a test agent.

### Propagation of Heart Cells

A heart cell culture can be obtained by allowing heart cells to migrate out of fragments of heart tissue adhering to a suitable substrate (e.g., a culture dish) or by disaggregating the tissue, e.g., mechanically or enzymatically to produce a suspension of heart cells. For example, the enzymes trypsin, collagenase, elastase, hyaluronidase, DNase, pronase, dispase, or various combinations thereof can be used. Trypsin and pronase give the most complete disaggregation but may damage the cells. Collagenase and dispase give a less complete dissagregation but are less harmful. Methods for isolating tissue (e.g., heart tissue) and the disaggregation of tissue to obtain cells (e.g., heart cells) are described in Freshney R. I., Culture of Animal Cells, A Manual of Basic Technique, Third Edition, 1994, the contents of which are incorporated herein by reference.

### Viral Vectors Suitable for Somatic Gene Transfer

Expression vectors, suitable for somatic gene transfer, can be used to express the compound, e.g., the phospholamban gene. Examples of such vectors include replication defective retroviral vectors, adenoviral vectors and adeno-associated viral vectors. Adenoviral vectors suitable for use by the methods of the invention include (Ad.RSV.lacZ), which includes the Rous sarcoma virus promoter and the lacZ reporter gene as well as (Ad.CMV.lacZ), which includes the cytomegalovirus promoter and the lacZ reporter gene. Methods for the preparation and use of viral vectors are described in WO 96/13597, WO 96/33281, WO 97/15679, and Trapnell et al., Curr. Opin. Biotechnol. 5(6):617-625, 1994, the contents of which are incorporated herein by reference.

### Expression of Phospholamban

The nucleic acid which results in the overexpression of phospholamban can be derived from the natural phospholamban gene including all the introns and exons, it can be a cDNA molecule derived from the natural gene (Fujji et al., J. Biol. Chem. 266:11669-11675, 1991, the contents of which are incorporated herein by reference) or a chemically synthesized cDNA molecule. The nucleic acid encoding the phospholamban protein can be under the control of the naturally occurring promoter or any other promoter that drives a high level expression of the phospholamban gene.

The following examples which further illustrate the invention should not be construed as limiting.

### Examples

### 1. Construction of E1-Deleted Recombinant Adenovirus Vectors

The construction of Ad.RSV.SERCA2a has been described in detail by Hajjar et al., *Circulation*, 95: 423-429, 1997, the contents of which are incorporated herein by reference. Ad.RSV.βgal, which carries a nuclear localizing form of β-galactosidase, is described in Dong et al., J. Biol. Chem. 27:29969-77, 1996, the contents of which are incorporated herein by reference. The rabbit phospholamban cDNA is described in Lylton J. MacLennan D.H., *J*. *Biol*. *Chem.,* 1988, 263:15024-15031, the contents of which are incorporated herein by reference. Briefly, the phospholamban cDNA was subcloned into the bacterial plasmid vector pAdRSV4, which uses the RSV long terminal repeat as a promoter and the SV40 polyadenylation signal and contains map units with adenovirus sequences from 0 to 1 and from 9 to 16. The position and orientation of the phospholamban cDNA were confirmed by restriction enzyme digestion and by polymerase chain reaction. The plasmid vector containing phospholamban (pAd.RSV-PL) was then cotransfected into 293 cells with PJM17. The homologous recombinants between pAd.RSV.PL and pJM17 contain the phospholamban cDNA substituted for El. By use of this strategy, independent plaques were isolated, and expression of phospholamban protein was verified by immunostaining. A positive plaque was further plaque-purified, and protein expression was reconfirmed to yield the recombinant adenovirus Ad.RSV.PL. This adenovirus is structurally similar to Ad.RSV.βgal and to Ad.RSV.SERCA2a, described in Dong O. et al., *J. Biol. Chem,* 1996, 271:29969-29977, 1976. The recombinant viruses were prepared as high-titer stocks by propagation in 293 cells as described in Graham, F.L. et al., *Methods in Molecular Biology: Gene Transfer and Expression Protocols,* 1991, 109-128, the contents of which are incorporated herein by reference. The titers of stocks used for these studies were as follows: 3.1x10¹⁴ pfu/mL for Ad.RSV.PL 2.⁶x10¹⁰ pfu/m: for Ad.RSV.SERCA2a, and 2.7x10¹⁴ pfu/mL for Ad.RSV.βgal, with a particle-to-pfu ratio of 40:1, 42:1, and 37:1, respectively.

### 2. Preparation of Neonatal Cardiomyocytes

Spontaneously beating cardiomyocytes were prepared from 1 to 2 day old rats and cultured in P-10 medium (GIBCO,BRL) in the presence of 5% fetal calf serum and 10% horse serum for 3 days as described previously in Kang J.X. et al., *Proc. Natl. Acad*. *Sci*. *U.S.A*., 1995, 92:3097-4001 and Kang J.X. and Leaf A., *Euro*. *J*. *Pharmacol*., 1996, 297:97-106, the contents of which are incorporated herein by reference. Measurements of cell shortening and cytosolic Ca²⁺ were performed on neonatal cardiomyocytes cultured on round, coated, glass coverslips (0.1 mm thickness, 31 mm diameter) in 35 mm culture dishes. Cells were counted using a hemocytometer. Approximately 5x10⁵ cells were plated in each coverslip.

### 3. Adenoviral Infection of Isolated Cells

In three different infection experiments with increasing concentrations of Ad.RSV.βgal, the percentages of cells expressing βgal after 48 hours, by histochemical staining in 10 different high-power fields were 98.2% (multiplicity of infection, 1 pfu/cell), 99.1% (multiplicity of infection, 10 pfu/cell), and 100% (multiplicity of infection, 100 pfu/cell). In a similar manner, myocardial cells were infected with three concentrations of Ad.RSV.PL 1.0, 10. and 100 pfu/cell for 48 hours. Infection with either Ad.RSV.βgal, Ad.RSV.PL, or Ad.RSV.SERCA2a did not change the morphology of the cells. For each infection experiment with the adenovirus, one myocyte was used to measure functional parameters. As shown in Figure 1, there was a 4-fold increase in phospholamban protein levels in a dose-dependent increase in the protein expression of phospholamban between 1 and 10 pfu/cell but no further increases between 10 and 100 pfu/cell. Coinfection of Ad.RSV.SERCA2a with Ad.RSV.PL produced an increase in protein expression of both SERCA2a and phospholamban, as shown by the immunoblot in Figure 2. There were no significant differences between the phospholamban protein levels in the group of myocytes infected with Ad.RSV.PL alone at a multiplicity of infection of 10 pfu/cell and the group of myocytes infected with Ad.RSV.PL at an multiplicity of infection of 10 pfu/cell and Ad.RSV.SERCA2a at a multiplicity of infection of 10 pfu/cell (*P*>2). Similarly, there were no significant differences between the SERCA2a protein levels in the group of myocytes infected with Ad.RSV.SERCA2a alone at a multiplicity of infection of 10 pfu/cell and the group of myocytes infected with Ad.RSV.PL at a multiplicity of infection of 10 pfu/cell and Ad.RSV.SERCA2a at an multiplicity of infection of 10 pfu/cell (*P*>2).

As shown in Figure 7, cardiomyocytes infected with Ad.RSV.PL (multiplicity of infection of 10 pfu/cell) exhibited a significant increase in resting Ca²⁺ not evident in uninfected cells. Furthermore, coinfection with Ad.RSV.SERCA2a (multiplicity of infection of 10 pfu/cell) restored the frequency response to normal.

The response to increasing stimulation frequencies in mammalian cardiomyocytes is governed by the SR. We have shown that in the uninfected cardiomyocytes, an increase in stimulation frequency did not significantly alter either peak or resting (Ca²⁺). This response is typical of rat cardiomyocytes that have either a flat response to increasing frequency of stimulation or a decrease in contractile force. However, in cardiomyocytes infected with Ad.RSV.PL, there was a significantly greater increase in resting (Ca²⁺) and a decrease in peak (Ca²⁺). These results would suggest that diminished SR Ca²⁺ uptake leads to a diminished CA²⁺ release, which becomes even more accentuated at higher frequencies of stimulation.

### 3. Intracellular Ca²⁺ Measurements and Cell Shortening Detection

Measurements of intracellular Ca²⁺ and cell shortening were performed as described earlier in Hajjar et al. (1997), Kang et al. (1995) and Kang et al (1996), the contents of which are incorporated herein by reference. Briefly, myocardial cells were loaded with the Ca²⁺ indicator fura 2 by incubating the cells in medium containing 2 µ mol/L fura 2-AM (Molecular Probex) for 30 minutes. The cells were then washed with PBS and allowed to equilibrate for 10 minutes in a light-sealed temperature-controlled chamber (32°C) mounted on a Zeiss Axlovers 10 inverted microscope (Zeiss). The coverslip was superfused with a HEPES-buffered solution at a rate of 20 mL/h. Cells were stimulated at different frequencies (0.1 to 2.0 Hz) using an external stimulator (Grass Instruments). A dual excitation spectrofluorometer (IONOPTIX) was used to record fluorescence emissions (505 nm) elicited from exciting wavelengths of 360 and 380 nm. [Ca²⁺] was calculated according to the following formula: [Ca²⁺]=*K*_{*d*} (R-Rₘᵢₙ*)*/(Rₘₐₓ -R)D, where R is the ratio of fluorescence of the cell at 360 and 380 nm: Rₘᵢₙ and Rₘₐₓ represent the ratios of fura 2 fluorescence in the presence of saturating amounts of Ca²⁺ and effectively "zero Ca²⁺ respectively, K_{d} is the dissociation constant of Ca²⁺ from fura 2; and D is the ratio of fluorescence of fura 2 at 380 nm in zero Ca²⁺ and saturating amounts of Ca²⁺. Unless otherwise stated, measurements of peak [Ca²⁺] were made at the end of diastole. High-contrast microspheres attached to the cell surface of the cardiomyocytes were imaged using a charge-coupled device video camera attached to the microscope, and motion along a selected raster line segment who quantified by a video motion detector system (IONOPTIX). As shown in Figure 4A, cardiomyocytes infected with Ad.RSV.βgal did not affect the Ca²⁺ transient or shortening compared with control uninfected cardiomyocytes. As depicted in Figure 4B, the Ca²⁺ transient and shortening were significantly altered with increasing concentrations of Ad.RSV.PL (multiplicity of infection of 1, 10, and 100 pfu/cell): observed changes included prolongation of the Ca²⁺ transient and shortening and a decrease in the peak Ca²⁺. These results, summarized in the following Table, show that there was a dose-dependent prolongation of the Ca²⁺ transient and mechanical shortening up to 10 pfu/cell, with no further significant prolongation at 100 pfu/cell, with no further significant prolongation at 100 pfu/cell.

| Physiological Parameters of Cardiomyocytes Overexpressing Phospholamban | | | | |
|---|---|---|---|---|
| | | | Ad.RSV.PL | |
| | Uninfected | MOI=1 pfu/Cell | MOI=10 pfu/Cell | MOI=100 pfu/Cell |
| Time to 80% relaxation of the (Ca2+) m? | 344±26 | 612±38° | 710±58 | 683±50 |
| Time to 80% relaxation of shortening, m? | 387±22 | 544±27° | 780±44 | 798±43 |
| Peak [Ca2+], µmol/L | 967±43 | 798±23° | 630±33 | 590±34 |
| n | 10 | 8 | 12 | 8 |

Similarly, peak [Ca²⁺] decreased up to 10 pfu/cell, with no further decrease at 100 pfu/cell. Coinfection with Ad.RSV.SERCA2a (multiplicity of infection of 10 pfu/cell) restored both the Ca²⁺ transient and the shortening to near normal levels, as shown in Figure 4C. Fig. 5 shows a significant decrease in mean peak [Ca²⁺], a significant increase in mean resting [Ca²⁺], and a significant prolongation of the Ca²⁺ transient in the group of cardiomyocytes infected with Ad.RSV.PL (multiplicity of infection of 10 pfu/cell) compared with uninfected cells (panels a through c, respectively). These effects were partially restored by the addition of Ad.RSV.SERCA2a (multiplicity of infection of 10 pfu/cell) (Figure 5). Similarly, the time course of shortening was significantly prolonged in cardiomyocytes infected with Ad.RSV.PL at a multiplicity of infection of 10 pfu/cell (time to 80% relaxation, from 387±22 to 780±44 milliseconds; *P* <0.5; n=12), whereas coinfection with Ad.RSV.SERCA2a restored the time course to normal (405±25 milliseconds, n=10, *P*>.1 compared with uninfected cells).

Adenoviral gene transfer of phospholamban provides an attractive system for further elucidation of the effects of inhibiting SR Ca²⁺-ATPase on intracellular Ca²⁺ handling. A decrease in SRCa²⁺ uptake rates is expected to lead to a smaller amount of Ca²⁺ sequestered by the SR, resulting in a smaller amount of Ca²⁺ release. In neonatal cardiomyocytes, a significantly prolonged Ca²⁺ transient and a higher resting (Ca²⁺) was observed reflecting the decreased Ca²⁴ uptake and a decrease in peak (Ca²⁺) levels reflecting less Ca²⁺ available for release. These results show that the SR Ca²⁴-ATPase is important during relaxation by controlling the rate and amount of CA²⁺ sequestered and during contraction by releasing the Ca²⁺ that is taken up by the SR. Overexpression of both phospholamban and SERCA2a partially restored the Ca²⁺ transient; however, the time course of the Ca²⁺ transient was still prolonged in cardiomyocytes infected with both Ad.RSV.SERCA2a and Ad.RSV.PL. This finding was somewhat surprising, since the SR Ca²⁺-ATPase activity was restored to normal and even enhanced in cardiomyocytes infected with both Ad.RSV.SERCA2a and Ad.RSV.PL.

Phospholamban has been shown to play a key role in modulating the response of agents that increase cAMP levels in cardiomyocytes. Since phosphorylation of phospholamban reduces the inhibition to the SR Ca²⁺ pump, thereby enhancing the SR Ca²⁺-ATPase, we were specifically interested in evaluating the effects of β-agonism on the relaxation phase of the Ca²⁺ transient. In the basal state, the overexpression of phospholamban significantly prolongs the Ca²⁺ transient. As shown in Figure 6, at maximal isoproterenol stimulation, the time course of the Ca²⁺ transients in the uninfected cardiomyocytes and the cardiomyocytes infected with Ad. RSV.PL were decreased to the same level. These findings show that phospholamban plays a major role in the enhanced relaxation of the heart to β-agonism. In addition, it corroborates these findings that phospholamban decreases the affinity of the SR Ca²⁺ pump for Ca²⁺ but does not decrease the maximal Ca²⁺ uptake rate.

### 4. Preparation of SR Membranes From Isolated Rat Cardiomyocytes

To isolate SR membrane from cultured cardiomyocyes, a procedure modified from Harigaya et al., *Circ. Res.,* 1969, 25:781-794, as well as, Wienzek et al., 1992, 23:1149-1163, the contents of which are incorporated herein by reference, was used. Briefly, isolated neonatal cardiomyocytes were suspended in a buffer containing (mmol/L) sucrose 500, phenylmerhyisulfonyl fluoride 1 and PIPES 20, at pH 7.4. The cardiomyocytes were then disrupted with a homogenizer. The homogenates were centrifuged at 500g for 20 minutes. The resultant supernatant was centrifuged at 25,000g for 60 minutes to pellet the SR-enriched membrane. The pellet was resuspended in a buffer containing (mmol/L) KCl 600, sucrose 30, and PIPES 20, frozen in liquid nitrogen, and stored at -70°C. Protein concentration was determined in these preparations by a modified Bradford procedure, described in Bradford et al., *Anal. Biochem*., 1976, 72:248-260, the contents of which are incorporated herein by reference, using bovine scrum albumin for the standard curve (Bio-Rad).

### 5. Western Blot Analysis of Phospholamban and SERCA2a in SR Preparations

SDS-PAGE was performed on the isolated membranes from cell cultures under reducing conditions on a 7.5% separation gel with a 4% stacking gel in a Miniprotean II cell (Bio-Rad). Proteins were then transferred to a Hybond-ECL nitrocellulose for 2 hours. The blots were blocked in 5% nonfat milk in Tris-buffered saline for 3 hours at room temperature. For immunoreaction, the blot was incubated with 1:2500 diluted monoclonal anti-SERCA2 antibody (Affinity BioReagents) or 1:2500 diluted anti-cardiac phospholamban monoclonal IgG (UBI) for 90 minutes at room temperature. After washing, the blots were incubated in a solution containing peroxidase-labeled goat anti-mouse IgG (dilution, 1:1000) for 90 minutes at room temperature. The blot was then incubated in a chemiluminescence system and exposed to an X-OMAT x-ray film (Fuji Films) for 1 minute. The densities of the bands were evaluated using NIH Image. Normalization was performed by dividing densitometric units of each membrane preparation by the protein amounts in each of these preparations. Serial dilution of the membrane preparations revealed a linear relationship between amounts of protein and the densities of the SERCA2a immunoreactive hands (data not shown).

### 6. SR Ca²⁺-ATPase Activity

SR Ca²⁺-ATPase activity assays were carried out according to Chu A. et al., *Methods Enzymol.,* 1988, 157:36-46, the contents of which are incorporated herein by reference, on the basis of pyruvate/NADH-coupled reactions. By use of a photomotor (Beckman DU 640) adjusted at a wavelength of 540 nm, oxidation of NADH (which is coupled to the SR Ca²⁺-ATPase) was assessed at 37°C in the membrane preparations by the difference of the total absorbance and basal absorbance. The reaction was carried out in a volume of 1 mL. All experiments were carried out in triplicate. The activity of the Ca²⁺-ATPase was calculated as follows: Δabsorbance/6.22 x protein x time (in nmol ATP/mg protein x min). The measurements were repeated at different [Ca²⁺] levels. The effect of the specific Ca²⁺-ATPase inhibitor CPA at a concentration range of 0.001 to 10 µmol/L was also studied in those preparations, as described in Schwinger et al., *Circulation*, 1995, 92:3220-3228 and Baudet et al., *Circ. Res.,* 1993, 73:813-819, the contents of which are incorporated herein by reference. As shown in Figure 3A, the relationship between ATPase activity and Ca²⁺ was shifted to the right in the preparations from cardiomyocytes overexpressing phospholamban compared with the uninfected preparations without changing maximal Ca²⁺-ATPase activity. Coinfection with Ad.RSV.SERCA2a restored the CA²⁺-ATPase activity and also increased the maximal Ca²⁺-ATPase activity. To verify that the ATPase activity measured from the membrane preparations was SR-related, the specific inhibitor CPA was used after maximally activating the SR Ca²⁺-ATPase with 10 µmol/L of Ca²⁺. As shown in Figure 3B, CPA inhibited the SR Ca²⁺-ATPase activity in a dose-dependent fashion in all three membrane preparations (uninfected, Ad.RSV.PL, and d.RSV.PL+Ad.RSV.SERCA2a).

The SR Ca²⁺-ATPase plays a key role in excitation-contraction coupling, lowering Ca²⁺ during relaxation in cardiomyocytes, and "loading" the SR with Ca²⁺ for the subsequent release and contractile activation. The Ca²⁺-pumping activity of this enzyme is influenced by phospholamban. In the unphosphorylated state, phospholamban inhibits the Ca²⁺-ATPase, whereas phosphorylation of phospholamban by cAMP-dependent protein kinase and by Ca²⁺ calmodulin-dependent protein kinase reverses this inhibition. Therefore, an increase in phospholamban content should decrease the affinity of the SR Ca²⁺ pump for Ca²⁺. As shown in Figure 4, overexpression of phospholamban shifted the relationship between SR Ca²⁺-ATPase activity and Ca²⁺ to the right, indicating a decrease of the sensitivity of the SR Ca²⁺ to pump to Ca2⁺. However, there was no change in the maximal Ca²⁺-ATPase activity in the Ad.RSV.PL-infected cardiomyocytes. This shows that the Vₘₐₓ of the Ca²⁺-ATPase of cardiac SR is not altered by interaction with phospholamban and phosphorylation, and that in mice overexpressing phospholamban, the affinity of the SR Ca²⁺ pump for Ca²⁺ was decreased but that the maximal velocity of the SR Ca²⁺ uptake was not changed. From the present experiment, it can also be concluded that phospholamban affects the affinity of the SR Ca²⁺ pump for CA²⁺ without changing the maximal ATPase activity. The concomitant overexpression of SERCA2a and phospholamban restored the ATPase activity and also increased the maximal Ca²⁺-ATPase activity. This brings further evidence that the expression of additional SR Ca²⁺-ATPase pumps can overcome the inhibitory effects of phospholamban.

### 7. Statistical Analyses

Data were represented as mean ± SEM for continuous variables. Stutent's test was used to compare the means of normally distributed continuous variables. Parametric one-way ANOVA techniques were used to compare normally distributed contiguous variables among uninfected groups of cells, Ad.RSV.βgal-infected cells, Ad.RSV.PL-infected cells, and Ad.RSV.SERCA2a-infected cells.

### 8. Retroviral Somatic Gene Transfer

Rats and mice were anesthetized with intraperitoneal pentobarbital and placed on a ventilator. The chest was entered form the left side through the third intercostal space. The pericardium was opened and a 7-0 suture placed at the apex of the left ventricle. The aorta and pulmonary artery were identified. A 22 G catheter containing 200 µl of adenovirus was advanced from the apex of the left ventricle to the aortic root. The aorta and pulmonary artery were clamped distal to the site of the catheter and the adenovirus solution was injected as shown in Figure 9. The clamp was maintained for 10 seconds while the heart was pumping against a closed system (isovolumically). This allowed the adenovirus solution to circulate down the coronary arteries and perfuse the whole heart without direct manipulation of the coronaries. After the 10 seconds, the clamp on the aorta and the pulmonary artery was released, the chest was evacuated from air and blood and closed. Finally, the animals were taken off the ventilator.

The expression pattern seen after direct injection is localized whereas the catheter-based technique is essentially homogeneous. The pressure tracing of the Ad.PL transduced hearts displayed a markedly prolonged relaxation and reduced pressure development as shown in Figure 8.

### 9. Gene Transfer of the Sarcoplasmic Reticulum Calcium ATPase Improves Left Ventricular Function in Aortic-Banded Rats in Transition to Failure

In human and experimental models of heart failure, sarcoplasmic reticulum Ca²⁺ ATPase (SERCA2a) activity has been shown to be significantly decreased. In this example the ability of SERCA2a expression to improve ventricular function in heart failure was investigated by creating an ascending aortic constriction in 10 rats. After 20-24 weeks, during the transition from left ventricular hypertrophy to failure, 200 µl of a solution containing 5x10⁹ plaque forming units of replication-deficient adenovirus carrying SERCA2a (Ad.SERCA) (n=4) or the reporter gene β-galctosidase (Ad.βgal) (n=6) were injected intracoronary via the catheter-based technique described *supra.* Two days after the procedure, the rats underwent open chest measurement of left ventricular pressure. Heart rate (HR), left ventricular end-diastolic pressure (LVEDP), and left ventricular systolic pressure (LVSP) were measured. Peak +dP/dt and -dP/dt were calculated. As shown in Table 1, the magnitudes of peak +dP/dt and -dP/dt which are indices of systolic and diastolic function were markedly increased in hearts transduced with the SERCA2a carrying adenovirus. Therefore, this example indicates that overexpression of SERCA2a in a rat model of pressure-overload hypertrophy in transition to failure *improved* left ventricular systolic and diastolic function.

**TABLE 1**

| | HR (bpm) | LVEDP (mmHg) | LVSP (mmHg) | +dP/dt (mmHg/sec) | -dP/dt (mmHg/sec) |
|---|---|---|---|---|---|
| Ad.βgal | 416 ± 46 | 6 ± 4 | 114 ± 16 | 5687 ± 1019 | -5023 ± 1803 |
| Ad.SERCA | 450 ± 53 | 9 ± 3 | 148 ± 40 | 9631 ± 3568^{#} | -8385 ± 980^{#} |

| | | | | | |
|---|---|---|---|---|---|
| # p<0.05 compared to Ad.βgal | | | | | |

### 10. Gene Transfer of Antisense of Phospholamban Improves Contractility in Isolated Cardiomyocytes

Delayed cardiac relaxation in failing hearts is attributed to a reduced activity of the Sarcoplasmic Reticulum Calcium ATPase. Phospholamban inhibits SERCA2a activity and is, therefore, a potential target to improve cardiac function. In this Example, an adenovirus carrying the full length antisense cDNA of phospholamban (Ad.asPL) was constructed using the methods described above. This construct was then used to infect neonatal cardiac myocytes as described in Example 3. As indicated in Figure 10A, infection of neonatal cardiac myocytes with the Ad.asPL construct increased the contraction amplitude and significantly shortened the time course of the contraction. The adenovirus-mediated gene transfer of the antisense cDNA for phospholamban also resulted in a modification of intracellular calcium handling and shortening in myocardial cells (see Figure 10B) indicating that such vectors can be used for increasing the contractility of myocardial cells in heart failure.

### Equivalents

Those skilled in the art will recognize, or be able to ascertain, using no more than routine experimentation, many equivalents to the specific embodiments of the invention described herein. Such equivalents are intended to be encompassed by the following claims:

## Claims

1. The use of a nucleic acid that directs expression of an SR Ca⁺² ATPase in the preparation of a medicament for a method of treating a heart disorder which method involves administering the nucleic acid.

2. The use of claim 1, wherein the method of administering comprises: restricting the aortic flow of blood out of the heart, such that blood flow is re-directed to the coronary arteries; introducing the nucleic acid into the lumen of the circulatory system such that it flows into the coronary arteries; allowing the heart to pump while the aortic outflow of blood is restricted; and re-establishing the flow of blood to the heart.

3. The use of claim 1, wherein the nucleic acid is contained in a viral vector suitable for somatic gene delivery.

4. The use acid of claim 3, wherein the viral vector is an adenoviral vector.

5. The use of claim 1, wherein the heart disorder is heart failure, ischemia, arrhythmia, myocardial infarction, congestive heart failure, or transplant rejection.
